# EUROPEAN PATENT APPLICATION

(11) **EP 2 436 759 A2**
(43) Date of publication of application: **04.04.2012**
(21) Application number: 10780829.7
(22) Date of filing: 28.05.2010
(51) Int. Cl.: C12N 5/04, C12N 5/02

(54) **PLANT STEM CELL DERIVED FROM CAMBIUM OF FAMILY SOLANACEAE, AND METHOD FOR ISOLATING AND CULTURING SAME**

(30) Priority: 28.05.2009 KR 20090047121
(71) Applicant: Unhwa Corporation, Jeollabuk-do 561-211 (KR)
(72) Inventor: YU, Young Mi, Jeonju-si Jeollabuk-do 560-879 (KR); KIM, So Yeon, Iksan-si Jeollabuk-do 570-764 (KR); JIN, Young Woo, Jeonju-si Jellabuk-do 561-808 (KR); LEE, Eun Kyong, Iksan-si Jeollabuk-do 570-976 (KR); HONG, Sun Mi, Jeonju-si Jeollabuk-do 561-231 (KR)
(74) Representative: Kiddle, Simon John
(86) International application number: PCT/KR2010/003424
(87) International publication number: WO 2010/137918

(57) **Abstract**

The present invention relates to a stem cell derived from a cambium of family *Solanaceae* and a method of isolating and culturing the same. The stem cell derived from the cambium of the family *Solanaceae* is isolated in an undifferentiated state without undergoing a dedifferentiation process, such that it can be stably maintained without variations in the growth rate and growth pattern thereof even during long-term culture, and thus can be cultured in large amounts. In addition, the disclosed stem cell and a culture medium thereof more efficiently inhibit the production of enzyme associated with the progression of aging compared to retinoic acid known to have a strong effect of reducing wrinkles, and they have the effect of inducing procollagen biosynthesis, and thus are useful for preventing and delaying aging.

## Description

### TECHNICAL FIELD

The present invention relates to a stem cell derived from a cambium of the family *Solanaceae* and a method of isolating and culturing the same.

### BACKGROUND ART

Plants were recognized as food resources in the past, but the meaning thereof is currently expanded to include a source for a wide range of chemical substances, including drugs, perfumes, pigments, agricultural chemicals and dyes and etc. Particularly, because most useful substances derived from plants have physiological activities, including antiviral, antibacterial, anticancer and antioxidant activities, plants are considered as ideal resources which can be developed into novel drugs, and active studies are in progress to elucidate the relationship between the chemical structures and activities of many plant-derived substances.

However, physiologically active substances are difficult to develop into drugs, and the main reasons therefore are as follows. First, the contents of physiologically active substances in plants are very limited. Second, the growth rate of plants is very slow. Third, physiologically active substances derived from plants are present only in specific organs of plants in small amounts. Fourth, environmental problems associated with the destruction of nature are involved. Fifth, physiologically active substances derived from plants have very complicated chemical structures, such that multi-step polymerization processes are required, thus causing economic problems of high production costs. For these reasons, it was very difficult to stably supply physiologically active substances derived from plants for commercialization.

Meanwhile, a plant cell culture method, one of bioengineering techniques, has been evaluated for a long time as the most ideal technique which can supply plant-derived useful substances without causing environmental problems. According to Korean Patent Publication No. 1995-0000870 (January 3, 1995), the production of useful substances by plant cell culture technique provides many advantages over methods of extracting useful substances directly from plants. Particularly, the plant cell culture method has been regarded as an optimal method which allows continuous production without being influenced by external environments so as to solve outstanding problems such as ecosystem destruction, unlike the prior extraction methods. However, despite great interest and effort in plant cell culture, examples which succeeded in industrializing the plant cell culture are still insufficient. This is because the variations in cell growth and productivity in a number of plant cell cultures still remain as a major problem.

In a case of that plant cells are used in plant expression systems, a dedifferentiation process is necessarily carried out in advance in order to convert tissues into a cell line having the ability to divide because the tissues which differentiated from the plant cells, for example, leaves, stems and seeds, are permanent tissues whose cells no longer divide. The dedifferentiation process means dedifferentiating tissues or cells, which have already been differentiated so as to perform specific functions, by culturing a plant tissue or organ . However, during this dedifferentiation process, serious changes in the cell line can occur due to chromosomal variations.

Particularly, the production of useful substances through plant cell culture can be industrialized, only when rapid cell growth and high metabolite productivity are stably maintained during a long-term culture period. However, most cells undergo many variations different from the original due to subculture. Thus, it is urgent to overcome this problem of variations in cells and to develop a method for acquiring a genetically stable cell line in producing useful substances through plant cell culture.

Korean Patent Registration 10-0533120 developed by some of the present inventors discloses a method of inducing callus using the cambium collected from the stem of a plant. However, in this registered patent, the callus is merely induced from the cambium of woody plant stem. This registered patent still has the problem of variation caused by dedifferentiation because the callus is a tissue formed through a dedifferentiation process.

Furthermore, some of the present inventors developed the invention of PCT/KR 2006/001544, which solves the problem of variation caused by dedifferentiation and relates to a method for providing cell lines that can stably proliferate and have high genetic stability. Meanwhile, tomato, a member of the genus *Lycopersicon* of the family *Solanaceae,* is widely known as a useful plant containing lycopene, tomatin, chlorophyll, vitamins A and C, minerals and organic acids and the like. As a result, there has been a need to obtain a cell line that overcomes the problem of variation caused by dedifferentiation, can stably proliferate and has high genetic stability.

Accordingly, the present inventors have made extensive efforts to obtain a useful plant cell line by isolating a stem cell derived from the cambium of family *Solanaceae.* As a result, the present inventors have isolated a stem cell derived from the cambium of a plant of the family *Solanaceae* and have found that the isolated stem cell stably proliferates and does not vary during culture, thereby completing the present invention.

### DISCLOSURE OF INVENTION

Therefore, it is an object of the present invention to provide a stem cell derived from a cambium of family *Solanaceae* without undergoing a dedifferentiation process, and a method of isolating the stem cell.

To achieve the above object, the present invention provides a stem cell derived from a cambium of family *Solanaceae,* in which the stem cell is an innately undifferentiated cell without going through dedifferentiation.

The present invention also provides a method of isolating a stem cell derived from a cambium of family *Solanaceae,* the method comprising the steps of:
(a) obtaining a tissue comprising a cambium from a plant of the family *Solanaceae*;
(b) culturing the obtained cambium- comprising tissue in a medium; and
(c) isolating a cell from the cambium, thereby obtaining a stem cell derived from the cambium.

Other features and embodiments of the present invention will be more apparent from the following detailed descriptions and the appended claims

### BRIEF DESCRIPTION OF THE DRAWINGS

FIG. 1A is a photograph showing a plant material (tomato), and FIG. 1B is a photograph showing that cambium-derived stem cells were induced and started to be separated from a callus layer derived from other tissues.
FIG. 2A is a micrograph showing the degree of aggregation of stem cells according to the present invention, and FIG. 2B is a micrograph showing the degree of aggregation of cells in a callus of tomato.
FIG. 3A is a photograph showing that a cambium-derived stem cell has a morphological feature, having numerous vacuoles, FIG. 3B is a photograph showing mitochondria in a cambium-derived stem cell, and FIG. 3C is a photograph showing mitochondria in the callus of tomato.
FIG. 4 is a graphic diagram showing the growth rates of stem cells according to the present invention and the callus of tomato.
FIG. 5 is a set of photographs showing the result of culture for 7 days in a 3-liter air-lift bioreactor (FIG. 5A), a 20-liter air-lift bioreactor (FIG. 5B) and a 250-liter air-lift bioreactor (FIG. 5C).
FIG. 6 is a set of photographs showing a tracheary element (TE) that differentiated from a stem cell according to the present invention, in which FIG. 6A is a polarized-light microscope photograph, and FIG. 6B is an optical microscope photograph (scale bar at the bottom of each photograph: 50 µm; magnification ×400).
FIG. 7 is a graphic diagram showing the stimulation of procollagen biosynthesis by each of a stem cell extract according to the present invention and a culture medium according to the present invention.
FIG. 8 is a graphic diagram showing the inhibitory effect of each of a stem cell extract and culture medium of the present invention on MMP-1 production induced by irradiation of fibroblasts with UV light.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein is well known and commonly employed in the art.

The definition of main terms used in the detailed description of the invention is as follows.

As used herein, the vascular "cambium" refers to a lateral meristem located within vascular tissue of a plant and is located on a stem and a root. The thickening growth of plants occurs by the activity of the cambium, and as a result, giant plants having more than 11,000 years of the growth rings may exist. Embryologically, the vascular cambium originates from the procambium, and thus is the same meristem which has been gradually differentiated with meristematic continuity (Jae-Doo LEE and seven others, "Plant Morphology", Academy Book Publishing Co, Chapter 10, 1993). In the present invention, the term "cambium" is meant to include a procambium. Such cambium and procambium are primary meristem, and thus in the present invention, it is obvious that the use of such cambium and procambium provides the same effect.

As used herein, the term plant "stem cell" refers to an innately undifferentiated cell without passing through a dedifferentiation process and is thus genetically highly stable.

As used herein, the term "innately undifferentiated" means that cells are not present in an undifferentiated state through a dedifferentiation process, but are originally maintained in a pre-differentiated state.

As used herein, the term "callus" refers to cells or cell clusters which have not differentiated through a dedifferentiation process (PNAS, 99(25):15843, 2002).

In one aspect, the present invention is directed to stem cell derived from a cambium of family *Solanaceae,* in which the stem cell is an innately undifferentiated cell without going through dedifferentiation.

When leaf, stem or root portions which are already differentiated tissues are used, they undergo a dedifferentiation process in which they are rejuvenated to their undifferentiated state in order to form a callus. During the dedifferentiation process, a mutation in somatic cells appears to cause cell unstability. Accordingly, the present inventors have conducted studies on a plant cell system showing little or no somaclonal variation, and as a result, have found that, when a cell line is derived specifically only from the meristem cambium, the ability of the metistem itself vigorously to divide can be used, and thus a cell line, which has no somaclonal variation and is genetically highly stable and physiologically uniform, can be derived without undergoing a dedifferentiation process. Based on this finding, a cambium-derived stem cell was isolated.

A stem cell derived from the cambium of the family *Solanaceae* according to the present invention may have at least one of the following characteristics: (a) including a greater number of single cells or including smaller-sized cell aggregates in suspension culture than a dedifferentiated callus of the family *Solanaceae*; (b) having a greater number of mitochondria than the dedifferentiated callus of the family *Solanaceae*; (c) being capable of growing faster and longer than the dedifferentiated callus of the family *Solanaceae*; and (d) having lower sensitivity to shear stress in a bioreactor than the dedifferentiated callus of the family *Solanaceae*; and (e) having the capability to differentiate into tracheary elements.

As used herein, the phrase "having a greater number of mitochondria" means having a number of mitochondria which is at least two times greater than the number of mitochondria in the dedifferentiated callus of the family *Solanaceae.* Herein, the stem cells according to the present invention are characterized by having mitochondria having increased activity compared to that of mitochondria in the dedifferentiated callus of the family *Solanaceae,* in which the phrase "having mitochondria having increased activity" means having mitochondria moving actively under a microscope. In addition, the present inventors have found that stem cells derived from the cambium of a plant of the family *Solanaceae* have the capacity to differentiate into tracheary elements. The cells derived from the cambium of the family *Solanaceae* according to the present invention have self-renewal ability and pluripotency which are the characteristics of plant stem cells, indicating that the cells according to the present invention are stem cells.

In the present invention, the stem cells may have at least two of the above characteristics (a) to (e), preferably at least three of the above characteristics (a) to (e), and more preferably at least four of the above characteristics (a) to (e). In addition, the stem cells according to the present invention may also have all of the above characteristics (a) to (e).

In addition, the stem cell line according to the present invention is characterized by having a large number of vacuoles when isolated. As used herein, the phrase "having a large number of vacuoles" means having a number of vacuoles which is at least two times greater than the number of vacuoles in the dedifferentiated callus of the family *Solanaceae.* In addition, the stem cells according to the present invention have smaller vacuoles than the dedifferentiated callus of the family *Solanaceae.*

The stem cell according to the present invention can be obtained by an isolation method comprising the steps of: (a) obtaining a tissue comprising a cambium from a plant of the family *Solanaceae*; (b) culturing the obtained cambium-comprising tissue in a medium; and (c) isolating a cell from the cambium, thereby obtaining a cambium-derived stem cell. Step (b) of the method according to the present invention may be performed by culturing the cambium-containing tissue to induce a cultured cambium proliferated from the cambium, and step (c) of the method may be performed by isolating the cultured cambium to obtain a cambium-derived stem cell. Step (b) of the method may be performed by culturing the cambium-comprising tissue in a medium containing auxin, in which auxin in the medium may be NAA (α-naphtalene acetic acid) or IAA (indole-3-acetic acid). Such auxin may be contained at a concentration of 1-5 mg/ℓ in the medium. Also, step (c) of the method may be performed by isolating the cultured cambium from an amorphous callus layer that proliferated from tissues other than the cambium, thereby obtaining the cambium-derived stem cell.

In one Example of the present invention, tomato cambium-derived stem cells were isolated from tomato, a member of the genus *Lycopersicon* of the family *Solanaceae.* Thus, the stem cells according to the present invention are preferably stem cells derived from the cambium of a plant of the genus *Lycopersicon,* and more preferably stem cells derived from the cambium of tomato.

In another Example of the present invention, it was confirmed that the tomato cambium-derived stem cell and a culture medium thereof not only promote procollagen biosynthesis but also have an excellent inhibitory effect on the production of collagenase (MMP-1) compared to retinoic acid known to have an excellent effect of reducing wrinkles, suggesting that the tomato cambium-derived stem cell line and a culture medium thereof have the effect of preventing and reducing wrinkles. Thus, the stem cell line according to the present invention and a culture medium thereof will be useful as an effective anti-aging composition.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Preparation of stem cells derived from a cambium of family Solanaceae

### 1-1: Preparation of plant material

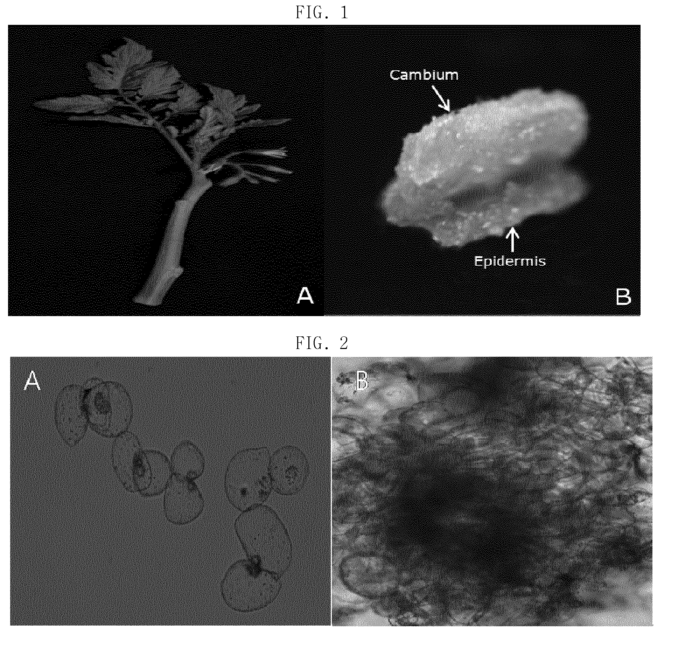

In order to obtain cambium-derived stem cells from tomato (*Lycopericum esculentum*; purchased from Sejong Seed Co., Ltd., Korea), a member of the genus *Lycopersicon* of the family *Solanaceae,* stems (FIG. 1) were collected from tomato, and then immediately soaked in 100 mg/L of the antioxidant L-ascorbic acid (DUCHEFA, The Netherlands). Then, they were transported and stored. Meanwhile, in order to obtain the same stem cells from the procambium of tomato, twigs in place of stems were collected.

Then, the plant was pretreated with a mixed solution of 0.1% benomyl (Dongbu Hannong Chemical, Korea), 0.1% daconil (Dongbu Hannong Chemical, Korea), 0.1% streptomycin sulphate (DUCHEFA, The Netherlands) and 0.01% cefotaxime sodium (DUCHEFA, The Netherlands) for 10 minutes, and then washed with tap water for 5 minutes to remove phenolic compounds and the remaining chemicals. Next, the plant was surface-sterilized in 70% ethanol (DC Chemical, Korea) for 1 min, 1.5% hydrogen peroxide (LG Chemical, Korea) for 3 min, 0.5% CLOROX solution for 5 min and 0.1% CLOROX solution for 5 min, and then washed 3-4 times with water.

### 1-2: Preparation of cambium-containing explants from stem and separation of tissue

The stem (twig when using the procambium), sterilized in Example 1-1 was cut and the phloem, cortex and epidermis tissues containing a cambium having an excellent ability to divide were peeled off from the xylem.

### 1-3: Induction of tomato cambium-derived stem cells

The cambium-containing explant prepared in Example 1-2 above was planted and cultured in the stem cell induction medium (medium 1) shown in Table 1 below.

**[Table 1]**

| Cell line induction medium (medium 1) | | |
|---|---|---|
| | Composition | Contents(mg/L) |
| Inorganic salts | KNO₃ | 2500 |
| | (NH₄)₂SO₄ | 134 |
| | MgSO₄·7H₂O | 121.56 |
| | MnSO₄·4H₂O | 10 |
| | ZnSO₄·7H₂O | 2 |
| | CuSO₄·5H₂O | 0.025 |
| | CaCl₂·2H₂O | 113.23 |
| | KI | 0.75 |
| | CoCl₂·6H₂O | 0.025 |
| | NaH₂PO₄·H₂O | 130.44 |
| | H₃BO₃ | 3 |
| | Na₂MoO₄·2H₂O | 0.25 |
| | FeNaEDTA | 36.7 |
| Vitamin | Myo-inositol | 200 |
| | Thiamine-HCl | 20 |
| | Nicotinic acid | 2 |
| | Pyridoxine-HCl | 2 |
| | L-ascorbic acid | 50 |
| | Citric acid | 75 |
| Amino acid | L-aspartic acid | 133 |
| | L-arginine | 175 |
| | Glycine | 75 |
| | Proline | 115 |
| Hormone | α-Naphtalene acetic acid | 2 |
| Sucrose | | 10,000 |
| Activated charcoal | | 100 |
| Gelrite | | 2,000 |

The growth regulator auxin such as NAA or IAA may be added to the medium at a concentration of 1-5 mg/L, and preferably 2 mg/L. The culture was carried out in a dark room controlled at 25±1 °C.

At 7-10 days of initial culture, the division of cells from the cambium was visually observed, and after 3 weeks (21 days) of culture, an amorphous callus formed by dedifferentiation started to be induced from the layer composed of phloem, cortex and epidermis. After 30 days of culture, the tissue started to be separated into the cultured cambium layer and the upper layer containing phloem, that is, an amorphous callus layer (FIG. 1B). After the tissue has been naturally completely separated into the two layers, the layers were separately cultured in different Petri dishes. After the tissue has been isolated, the white and soft portion thereof having good growth rate was subcultured in the same fresh medium as induction medium at an interval of 14 days.

For comparison, an explant was obtained from the tomato stem and sterilized, and then cultured in the medium of Table 1 to induce a callus. The callus derived from the stem explant had an irregular shape due to the difference in division rate between different cells, showed unstable growth rate and readily turned brown. However, the tomato cambium-derived stem cells maintained a bright color.

Also, the tomato cambium-derived stem cells were stably maintained without variations in their growth rates, growth patterns and aggregation degrees when they were cultured for a long period, confirming that the large scale cell culture would be possible. However, callus derived from the tomato stem showed variation in their growth pattern and growth rate and a high degree of aggregation when they were cultured for a long period of time. Thus, the callus cells tuned brown, were necrotized and could not be stably in a large scale.

### Example 2: Proliferation of stem cells derived from cambium of family Solanaceae and observation of characteristics of the stem cells

The cambium-derived stem cells isolated in Example 1 above were placed in a flask containing the liquid medium shown in Table 2 below. Then, the stem cells in the flask were cultured in a rotating shaker under dark conditions at 100 rpm at 25 ±1 °C. The interval of subculture was set at 2 weeks, such that the cultured cells could always be maintained in high activity in the exponential growth phase.

**[Table 2]**

| Suspension medium (medium 2) | | |
|---|---|---|
| | Composition | Contents(mg/L) |
| Inorganic salts | Ca(No₃)₂ | 471.26 |
| | NH₄NO₃ | 400 |
| | MgSO₄· 7H₂O | 180.54 |
| | MnSO₄· 4H₂O | 22.3 |
| | ZnSO₄· 7H₂O | 8.6 |
| | CuSO₄· 5H₂O | 0.25 |
| | CaCl₂· 2H₂O | 72.5 |
| | K₂SO₄ | 990 |
| | Na₂MoO₄· 2H₂O | 0.25 |
| | H₃BO₃ | 6.2 |
| | KH₂PO₄ | 170 |
| | FeNaEDTA | 36.7 |
| Vitamin | Myo-inositol | 200 |
| | Thiamine-HCl | 20 |
| | Nicotinic acid | 2 |
| | Pyridoxine-HCl | 2 |
| | L-ascorbic acid | 50 |
| | Citric acid | 75 |
| Amino acid | L-aspartic acid | 133 |
| | L-arginine | 175 |
| | Glycine | 75 |
| | Proline | 115 |
| Hormone | α-Naphtalene acetic acid | 2 |
| Sucrose | | 30,000 |

The degree of aggregation of the cells was observed with biological microscope CX31 (Olympus, Japan). As a result, as shown in FIG. 2A, it was observed that the stem cells according to the present invention included a large number of single cells during suspension culture, some of the cells were present as small-sized cell aggregates. Namely, when the stem cells according to the present invention were cultured, the maximum size of the cell aggregates was only 500 µm. On the contrary, in a control group, when the tomato callus (PC10623) supplied from the Korea Biological Resource Center was observed, and as a result, the callus cells were highly aggregated as shown in FIG. 2B, and the maximum size of the cell aggregates was 10 mm. In addition, the stem cells according to the present invention and the cells of the callus (PC10623) were sampled after the proliferative culture, but before subculture, and the cell viability (%) of the samples were calculated using a 2% Evan's blue staining (5min) method. As a result, as shown in Table 3, the stem cells according to the present invention showed a viability of 96.33%, whereas the callus cells showed a viability of 65.2%.

**[Table 3]**

| Cell lines | Aggregate size (µm) (Maximum size) | Survival rate (%) |
|---|---|---|
| Stem cell | 500 | 96.33 |
| Callus | 10000 | 65.2 |

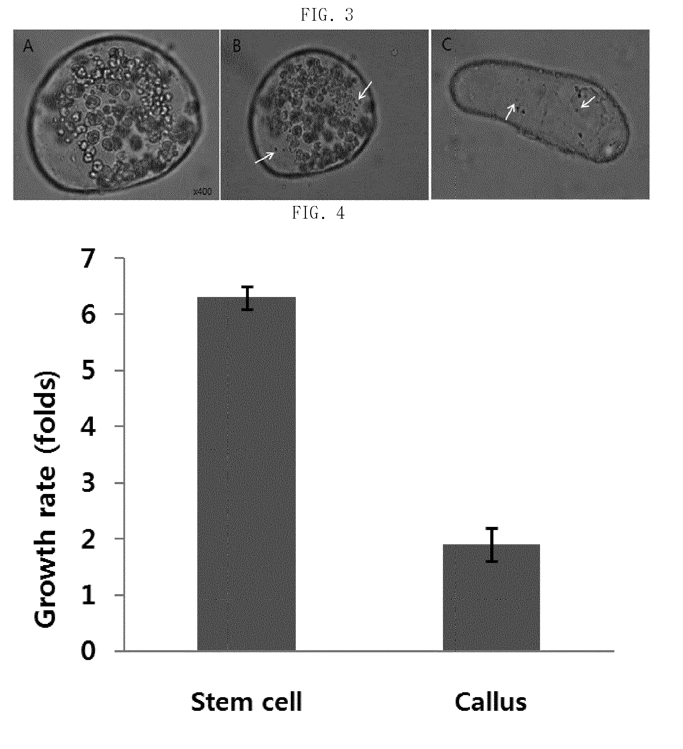

Meanwhile, the stem cell according to the present invention is morphologically characterized by a number of vacuoles, as shown in FIG. 3A. Specifically, the stem cell of the present invention is **characterized in that** it has a number of vacuoles immediately after isolation. However, it was found that, as pressure applied to stem cells during a culture process decreased, the isolated and cultured stem cells changed to a morphology having only 1 or 2 vacuoles. Such stem cells that changed to a morphology having a small number of vacuoles show a characteristic in that they will have a large number of vacuoles when pressure is applied thereto. This characteristic also appears in undifferentiated cells in plants by factors such as pressure, suggesting that the stem cells according to the present invention are in an undifferentiated state. Meanwhile, the stem cells according to the present invention were observed with optical microscope BX41TF. As a result, a large number of mitochondria having a very high activity in terms of movement could be observed in the stem cell. FIG. 3B shows that the stem cell according to the present invention has a large number of mitochondria. In FIG. 3B, the arrows indicate mitochondria. However, as shown in FIG. 3C, this characteristic could not be observed in the cells of the tomato callus (PC10623). In FIG. 3C, the arrows indicate mitochondria in the callus cells.

Meanwhile, in order to examine the possibility of large scale cell culture, the tomato cambium-derived stem cells according to the present invention were cultured in an airlift bioreactor (Samsung Science Co., Ltd., Korea) having an internal volume of 3 L. The culture was carried out in the liquid medium of Table 2 under dark conditions at 25±1 °C and an aeration rate of 0.1-0.3 vvm.

**[Table 4]**

| Results of measurement of growth rate and GI | | |
|---|---|---|
| Cell lines | Growth rate (folds) | GI |
| Stem Cell | 6.3 | 5.32 |
| Callus | 1.9 | 0.9 |

As a result, as shown in Table 4 above and FIG. 4, the growth rate of the tomato callus (PC10623) was 1.9 fold and the GI (growth index = (maximum DCW - initial DCW) / initial DCW) thereof was 0.9, but the cambium-derived stem cells according to the present invention had a growth rate of 6.3 fold and a GI of 5.32, which were higher those of the tomato callus. In the case of conventional reactors, as can be seen from the cell viability of the tomato callus in Table 4, cell viability rapidly decreases due to growth ring production in the reactor, plant cell aggregation during culture, and the sensitivity of hard cell walls to shear stress. However, the cambium-derived stem cells formed a very small growth ring area in the bioreactor during culture, and the ring formed on the internal wall thereof was simply eliminated when a simple stimulus was applied to the incubator. Also, it was shown that the cambium-derived stem cells had low aggregation and contained a large number of vacuoles, and thus had low sensitivity to shear stress, such that cell viability was not greatly affected by shear stress.

**[Table 5]**

| Large-scale culture process | | |
|---|---|---|
| | Bioreactors | Max. dry cell weight (g/L) |
| A | 3L air-lift bioreactor | 10.7 |
| B | 20L air-lift bioreactor | 11.6 |
| C | 250L air-lift bioreactor | 12.6 |

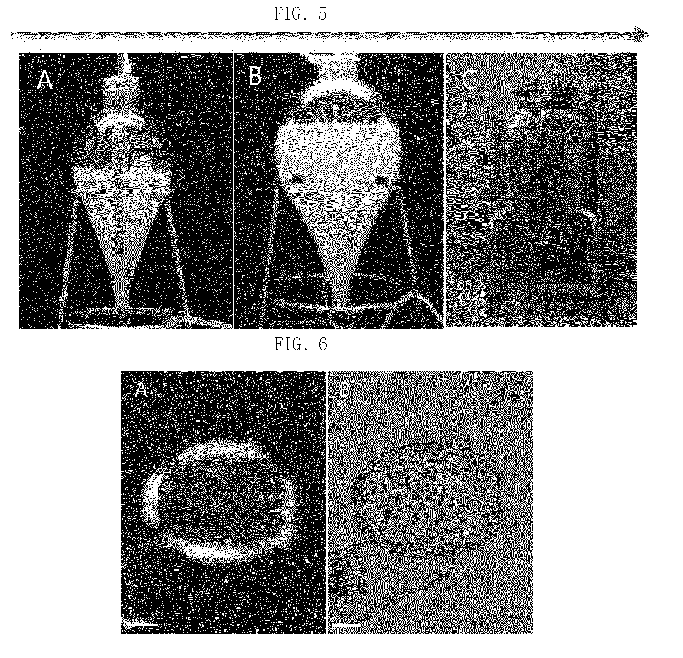

In addition, as can be seen in Table 5 and FIG. 5, when the stem cells were cultured in each of a 3-L air-lift bioreactor, a 20-L air-lift bioreactor and the 250-L air-lift bioreactor for 7 days, it was shown that the amount of dry cells per liter in the 250-L bioreactor were rather increased.

In other words, it was seen that the cambium-derived cells according to the present invention had low sensitivity to shear stress in the bioreactor for large scale culture, and thus could be produced rapidly in large amounts in the bioreactor. Accordingly, it could be seen that the cambium-derived stem cells according to the present invention had lower sensitivity to shear stress than cell derived from the dedifferentiated callus of tomato.

In addition, the characteristics of plant stem cells include self-renewal ability and pluripotency. Thus, in order to induce the tomato cambium-derived stem cells to differentiate into tracheary elements, the stem cells were cultured in each of MS media containing 10 mg/L NAA, 2 mg/L kinetin, 6 mg/L GA₃ and 6% sucrose at 25±1 °C under dark conditions. As a result, as can be seen in FIG. 6, the cambium-derived stem cells differentiated into tracheary elements.

### Example 3: Preparation of extract of stem cells derived from cambium of plant of family Solanaceae

The stem cells, which have been suspension-cultured for 14 days as described in Example 2, were collected, and the culture medium was removed therefrom. 500ml of DMSO were added to 500g of the obtained stem cells and it was stirred at 50°C for 6 hours for dissuolution. Then, the cell solution was centrifuged at 3,000 g for 10 minutes, and the supernatant was collected, thereby obtaining a DMSO soluble substance. The obtained DMSO soluble substance was concentrated using a rotary vacuum concentrator, and the concentrated sample was dried using a freeze dryer, thereby obtaining a DMSO extract of the stem cells.

### Example 4: Examination of promotion of procollagen biosynthesis - examination of anti-aging effect

Because a decrease in the production of collagen in the skin causes wrinkle production, the following test was carried out in order to examine whether the stem cells according to the present invention and a culture medium thereof promote procollagen biosynthesis. Normal human dermal fibroblasts (NHDFs) were purchased from MCTT Co., Ltd. (Korea) and cultured in 10% FBS-containing DMEM medium (Welgene, Korea).

First, fibroblasts were seeded in a 48-well plate at a density of 1 x 10⁴ cells/well and cultured. Then, each of samples was diluted in serum-free DMEM medium, and the cells were diluted with the dilution of each sample and cultured for 72 hours. Then, the amount of procollagen in the culture medium of fibroblasts was measured using a procollagen Type I C-peptide EIA kit (Takara Bio Inc., MK101) according to the manufacturer's instruction. As the test groups, 50 ppm of the DMSO extract of the tomato cambium-derived stem cells and 10 vol% of the culture medium of the stem cells were used, and as a positive control, 10 ng/mℓ of TGF-beta was used.

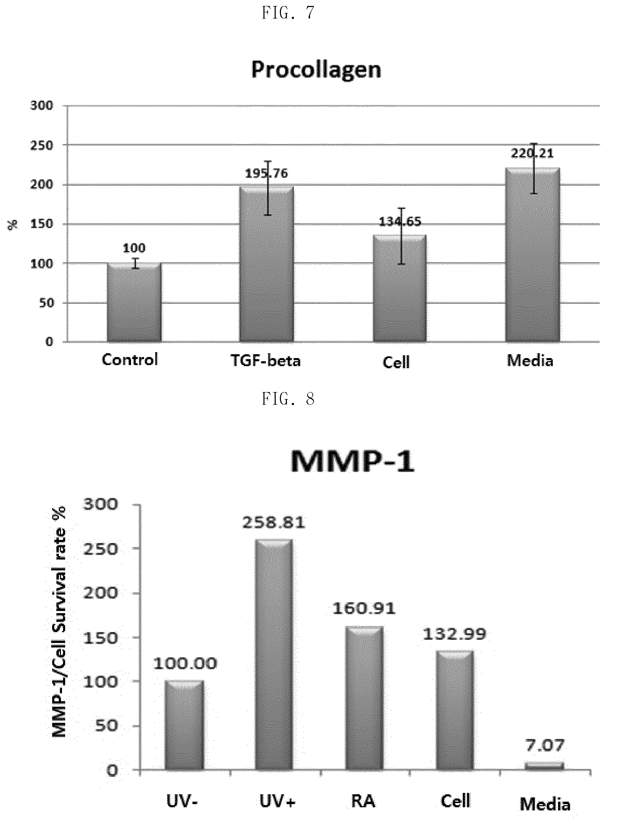

The measurement results were calculated relative to 100 for the amount of biosynthesized procollagen in a control. As a result, as can be seen in FIG. 7, both treatment with the stem cell extract and its culture medium according to the present invention showed the effect of promoting procollagen biosynthesis. Particularly, the culture medium of the stem cell showed an excellent effect of promoting procollagen biosynthesis compared to the positive control, TGF-beta and was thus expected to have an excellent effect of reducing wrinkles. In FIG. 7, "cell" indicates the DMSO extract of stem cells, and "culture medium" indicates the culture medium removed during the preparation of the stem cell extract.

### Example 5: Examination of inhibitory effect on collagenase (MMP-1) production induced by UV irradiation - examination of anti-aging effect

In case of the increase in MMPs by exposure to UV radiation, the increased MMPs degrade skin collagen to form wrinkles. Thus, the following test was carried out in order to test whether MMP-1 expression which increased by UV radiation is inhibited by the tomato cambium-derived stem cell extract or the culture medium of the stem cells.

First, fibroblasts (MCTT Co., Ltd., Korea) were seeded in a 24-well plate at a density of 3×10⁴ cells/well and cultured to adhere for 12 hours, then the cells were starved in FBS-free medium for 12 hours. Then, the cells were washed with DPBS buffer, irradiated with 10 mJ/cm² of UVA (365 nm) and treated with varying concentrations of each of samples (50 ppm of the DMSO extract of the tomato cambium-derived stem cells and 10 vol% of the culture medium) in serum-free DMEM medium for 48 hours. The culture media were collected and the amount of MMP-1 in the media was measured by Western blot assay and then ELISA assay. As a positive control, 10 µM of retinoic acid was used.

As a result, as shown in FIG. 8, the stem cell extract and its culture medium of the present invention effectively inhibited the expression of MMP-1 compared to retinoic acid known to have a strong effect of reducing wrinkles, indicating that they have the effect of preventing and reducing wrinkles. Particularly, the activity of MMP-1 in the cells treated with the culture medium was only 7.07 relative to 100 for that in the cells not treated with UV light, indicating that the culture medium has a very excellent effect on the inhibition of MMP-1 expression. This ability of the culture medium to inhibit MMP-1 expression was about 23 times higher than that of retinoic acid, suggesting that the culture medium is very useful as an anti-aging cosmetic composition.

Therefore, the test results of Example 6 together with Example 5 indicate that the stem cell extract and culture medium of the present invention have excellent anti-aging effects.

### INDUSTRIAL APPLICABILITY

As described above, the inventive stem cell line derived from the cambium of a plant of the family *Solanaceae* is isolated in an undifferentiated state without undergoing a dedifferentiation process, such that it can be stably maintained without variations in the growth rate and growth pattern thereof even during long-term culture, and thus can be cultured in large amounts. In addition, the disclosed stem cell and a culture medium thereof more efficiently inhibit the production of enzyme associated with the progression of aging compared to retinoic acid known to have a strong effect of reducing wrinkles, and they have the effect of inducing procollagen biosynthesis, and thus are useful for preventing and delaying aging.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A stem cell derived from a cambium of family *Solanaceae,* which is an innately undifferentiated cell without going through dedifferentiation.

2. The stem cell according to claim 1, wherein the stem cell additionally comprises at least one of the following characteristics:
(a) including a greater number of single cells or including smaller-sized cell aggregates in suspension culture than a dedifferentiated callus of the family *Solanaceae*;
(b) having a greater number of mitochondria than the dedifferentiated callus of the family *Solanaceae*;
(c) being capable of growing faster and longer than the dedifferentiated callus of the family *Solanaceae*;
(d) having lower sensitivity to shear stress in a bioreactor than the dedifferentiated callus of the family *Solanaceae*; and
(e) having the capability to differentiate into tracheary elements.

3. The stem cell according to claim 2, wherein the stem cell comprises two or more characteristics selected from the group consisting of the characteristics (a) to (e).

4. The stem cell according to claim 2, wherein the stem cell comprises three or more characteristics selected from the group consisting of the characteristics (a) to (e).

5. The stem cell according to claim 2, wherein the stem cell comprises four or more characteristics selected from the group consisting of the characteristics (a) to (e).

6. The stem cell according to claim 2, wherein the stem cell comprises the characteristics (a) to (e).

7. The stem cell according to claim 1, wherein stem cell is obtained by an isolation method comprising the steps of:
(a) obtaining a tissue comprising a cambium from a plant of the family *Solanaceae*;
(b) culturing the obtained cambium-comprising tissue in a medium; and
(c) isolating a cell from the cambium, thereby obtaining a stem cell derived from the cambium.

8. The stem cell according to any one claim among claims 1 to 7, wherein the plant of the family *Solanaceae* is genus *Lycopersicon.*

9. The stem cell according to any one claim among claims 1 to 7, wherein the plant of the family *Solanaceae* is tomato.

10. A method of isolating a stem cell derived from a cambium of family *Solanaceae,* the method comprising the steps of:
(a) obtaining a tissue comprising a cambium from a plant of the family *Solanaceae*;
(b) culturing the obtained cambium-comprising tissue in a medium; and
(c) isolating a cell from the cambium, thereby obtaining a stem cell derived from the cambium.

11. The method according to claim 10, wherein the obtained cambium-comprising tissue are cultured in the medium comprising auxin in the step (b).

12. The method according to claim 11, wherein the medium comprises 1∼5mg/L of auxin.

13. The method according to claim 10, wherein the step (c) comprises isolating cells from the cambium after separating the cultured cambium from an amorphous callus layer derived from tissues other than the cambium, thereby obtaining the stem cell derived from the cambium.

14. The method according to claim 10, wherein the plant of the family *Solanaceae* is genus *Lycopersicon.*

15. The method according to claim 10, wherein the plant of the family *Solanaceae* is tomato.
